# EUROPEAN PATENT APPLICATION

(11) **EP 4 483 920 A1**
(43) Date of publication of application: **01.01.2025**
(21) Application number: 23382652.8
(22) Date of filing: 26.06.2023
(51) Int. Cl.: A61M 5/14, A61M 5/162, A61M 39/22, A61M 5/168, A61M 5/36, A61M 39/26, A61M 39/00

(54) **FLUSHING SYSTEM FOR INFUSION LINE**

(71) Applicant: Universidad del País Vasco/Euskal Herriko Unibertsitatea, 48940 Leoia, Bikaia (ES)
(72) Inventor: AMPUDIA GOMEZ, Maria Arantzazu, E-48940 Leioa, Vizcaya (ES)
(74) Representative: ABG Intellectual Property Law, S.L.

(57) **Abstract**

The present invention relates to a medical device for the dosed administration of a liquid drug by means of gravity infusion. In particular, the invention is a gravity infusion device which allows optimizing drug delivery in intravenous therapies for patients undergoing treatment.

## Description

### OBJECT OF THE INVENTION

The present invention relates to a medical device for the dosed administration of a liquid drug by means of gravity infusion. In particular, the invention is a gravity infusion device which allows optimizing drug delivery in intravenous therapies for patients undergoing treatment. This device helps to drastically reduce the presence of highly contaminating substances in the environment.

### BACKGROUND OF THE INVENTION

Known infusion systems store the liquid drug to be administered in a container or vessel. To administer the drug in the human or animal body of a patient, infusion systems feature connecting equipment, known as saline equipment, which acts like fluidic connecting link between the container storing the drug and the patient.

Known saline equipment includes at a first end a piercing device connecting with the container storing the liquid drug, a drip chamber which allows the drip rate of the liquid drug coming from the container to be observed, and next a main tubing or conduit through which the liquid drug flows until being administered in the body of the patient, the main conduit including a flow regulator.

In practice, once the administration of a specific drug to a patient has ended, with the container thereby being left empty, part of this drug is still housed in the tubing of the saline equipment, without being administered to the patient. In other words, once the container containing the liquid drug is emptied, the infusion is considered to have ended, with traces of drug remaining in the saline equipment. Furthermore, once the infusion has ended, the whole infusion equipment is discarded, including part of the drug that has not been administered to the patient and ends up being potentially contaminating waste.

Therefore, it has been found that, in known infusion systems, once the infusion has ended, there is a problem and it is that a certain amount of drug is left housed therein without being infused into the patient. That is, about 12-15 ml of drug are discarded together with the infusion system once the infusion has ended. Not only is said amount of drug being discarded and the economic loss that this represents, but in addition, patients are not receiving the full drug dose that has previously been prescribed.

In view of these problems, the invention proposes the objective of optimizing the residual medication in infusion systems to ensure that the entire amount of medication prescribed for a patient is administered, thereby preventing the wasting of part of the medication as traces of same are left in the infusion device without being administered to the patient.

### DESCRIPTION OF THE INVENTION

The invention solves the aforementioned problem by means of an infusion device according to claim 1. Preferred embodiments of the invention are defined in the dependent claims.

In a first inventive aspect, the present invention presents a *gravity infusion device suitable for the intravenous infusion of a first fluid.*

The invention starts from a device for the dosed infusion by gravity of a first fluid into a patient, in which this first fluid is initially contained in a storage element or container from where it is delivered to the infusion device through an outlet of the storage element for the dosed administration thereof.

Patient or client will be understood to mean that human or animal individual that receives therapy.

In a preferred embodiment, *the first fluid is preferablya drug.* More particularly, the first fluid is a liquid drug or a drug that has been diluted in an excipient such as saline.

The device further comprises:
- *a main conduit, with a first end intended for being fed by a storage element for storing the first fluid to be infused and a second end with connection means to be connected to a line for delivering, in the operative mode, the first fluid to a patient.*

The main conduit will be understood to mean the tubing connecting at the ends thereof with the storage element for storing the first fluid and with the patient's line, respectively. In other words, this main conduit allows the fluidic communication between the storage element and the patient so as to be able to infuse the first fluid in the operative mode. In particular, the main conduit comprises at its second end connecting with the line connection means suitable for being connected to said line and thus allowing the delivery of the first fluid to the patient.

In the operative mode, the infusion device is first connected at the first end of the main conduit to the storage element for storing the first fluid and at the second end of the main conduit to the line through which said first fluid will be delivered to the patient.

Additionally, the device comprises:
- *a drip chamber interposed in a section of the main conduit proximal to the first end.*

The drip chamber is configured to continuously see the drip rate of the first fluid which, in the operative mode, will be delivered to the patient. This drip chamber is interposed in the main conduit, preferably in a place closer to the first end of this conduit than to the second end, that is, closer to the storage element from which the first fluid is delivered. Furthermore, the top part of the drip chamber is rigid while the lower part of the drip chamber is flexible. The indication of top and bottom are to be interpreted according to the direction of the action of gravity, and for the device, top refers to the relative position when the device is spread out in the operative mode, with the storage element therefore being in the upper part and the rest of the elements hanging right below it until reaching the patient. Even though the tubing may bend at any time, the positional correspondences will always be with respect to a spread-out and unbent tubing, maintaining the direction of the action of the gravity.

Additionally, the device comprises:
- *a storage and propulsion means for storing and propelling a pre-established amount of a second fluid, preferably saline solution for intravenous use, comprising a first fluidic connection point.*

The storage and propulsion means are configured for delivering, in the operative mode, a second fluid, different from the first fluid, the objective being to flush the first fluid from the device to thereby reduce the trace amount of the first fluid once the infusion has ended. These storage and propulsion means house a pre-established amount of a second fluid which is preferably a saline solution for intravenous use. Furthermore, the storage and propulsion means comprise a first connection point through which, in the operative mode, the second fluid is delivered in the infusion device. In particular, the saline solution which is administered in the infusion device is of a pre-established amount which coincides with or approximates the amount of fluid that would be necessary to reach the second end of the main conduit and thereby ensure that virtually all of the first fluid is administered to the patient, and preferably the fluid that is not the drug does not get to the patient.

Preferably, the second fluid is a saline solution for intravenous use suitable for the flushing of peripheral lines.

The device further comprises:
- *a three-way cock interposed in a section of the main conduit and located between the drip chamber and the second end, wherein the three-way cock comprises:*
   ∘ *a first inlet port fluidically connected with the drip chamber;*
   ∘ *a second inlet port further comprising*
      ▪ *a second fluidic connection point; and*
      ▪ *a biosafety valve, that is, the valve is open if the second port is fluidically connected with the first fluidic connection point and closed if it is not connected, the biosafety valve being placed before the second fluidic connection point and configured to allow the entry of the second fluid from the storage and propulsion means to the three-way cock;*
   ∘ *a third outlet port fluidically connected with a section of the main conduit extending up to the second end.*

The three-way cock is also interposed in the main conduit and in this case is positioned after the drip chamber between it and the second end of the main conduit. The three-way cock is in fluidic communication with the drip chamber at a first inlet port, with the storage and propulsion means at a second inlet port and with the second end of the main conduit at a third outlet port.

According to two alternatives, the first inlet port of the three-way cock can either be connected directly with the drip chamber or else with a section of the main conduit which in turns connects with the drip chamber. In the operative mode, it is through this first inlet port where the first fluid coming from the drip chamber flows to the three-way cock.

In the operative mode, it is through the second inlet port of the three-way cock where the second fluid coming from the storage and propulsion means enters the three-way cock. In particular, this second inlet port includes a second fluidic connection point and a biosafety valve arranged between the three-way cock and the second connection point. When the first connection point of the storage and propulsion means is connected with the second connection means of the second inlet port of the three-way cock, the biosafety valve is open, while when there is no connection between said connection points between the storage and propulsion means and the second inlet port, the biosafety valve remains closed, that is, it does not allow the passage of any fluid into or out of the three-way cock.

The third outlet port of the three-way cock is connected directly with the main conduit, in particular, with a section of the main conduit extending up to the second end of the main conduit. Therefore, in the operative mode, it is through this third outlet port where both the first fluid and the second fluid exit to the second end of the main conduit.

Additionally,
*a) the three-way cock comprises two operative positions:*
   *a first operative position in which the first port and the third port are fluidically communicated while the second port is fluidically closed, and*
   *a second operative position in which the first port is fluidically closed, and the second port and the third port are fluidically communicated.*

The first operative position is the one that allows the passage of the first fluid through the three-way cock to the second end of the main conduit. Specifically, in this first operative position, the first inlet port and the third outlet port of the three-way cock are in fluidic communication while the second inlet port is closed and does not allow the passage of any fluid through same.

The second operative position is the one that allows the passage of the second fluid through the three-way cock to the second end of the main conduit. In this position, the second inlet port and the third outlet port are in fluidic communication while the first inlet port is closed without allowing the passage of any fluid through same.

Lastly, in the device:
*b) the first fluidic connection point is configured to be coupled to the second fluidic connection point such that, in the operative mode, the propulsion of the second fluid from the storage and propulsion means displaces at least part of the first fluid stored in the section of the main conduit arranged between the three-way cock and the second end.*

In the operative mode, and with the three-way cock being in the second operative position, upon connecting the first fluidic connection point with the second fluidic connection point, the second fluid housed in the storage and propulsion means is delivered to the three-way cock such that this second fluid pushes the first fluid that is housed in the section of the main conduit between the three-way cock and the second end to the second end.

Therefore, the administration of a second fluid in the present infusion device achieves there being no traces of the first fluid or medication between the three-way cock and the second end once the infusion has ended. Advantageously, the waste of medication once the infusion has ended is considerably reduced, and the infusion of the full dose of medication that had previously been prescribed is thereby achieved.

In a particular embodiment, *the first end is a perforating element configured to be inserted into the storage element which is preferably a bottle or bag of the first fluid intended for being infused in a patient.*

In a more particular embodiment, the perforating element is a piercing device which is inserted into the storage element or container to fluidically connect the infusion device with said storage element for storing the first fluid or medication. In the instant that the piercing device is inserted into the bottle or bag, for example, in an access port thereof, the first fluid starts to exit this bottle or bag and flows through the main conduit.

In a particular embodiment, *the three-way cock comprises three wings providing with one another a T shape, and where the wings having an orientation coinciding with the orientation of a port of connection indicate the opening of said port.*

In the first operative position of the three-way cock, the two wings having a coinciding orientation are each respectively located in the first inlet port and in the third outlet port, thereby allowing the passage of the first fluid from the drip chamber to the second end of the main conduit. In other words, both first inlet port and third outlet port are open to allow said fluidic communication between the drip chamber and the second end, blocking the second inlet port of the three-way cock as no wing is positioned at said inlet and, furthermore, there is a biosafety valve preventing the passage of any fluid.

Moreover, in the second operative position of the three-way cock, the wings forming a T-shaped configuration are turned to open the second inlet port of the three-way cock and close the first inlet port to prevent the second fluid from flowing back to the storage bag of the first fluid. By means of the opening of this second inlet port, the passage of the second fluid from the storage and propulsion means to the second end of the main conduit, passing through the three-way cock, is allowed. After operating the three-way cock for the wings to rotate (with the three-way cock transitioning from the first operative position to the second operative position), the second inlet port and the third outlet port are open, allowing the fluidic communication between the storage and propulsion means and the second end of the main conduit.

In a particular embodiment, *the first fluidic connection point and second fluidic connection point are Luer lock type fluidic connection points* (https://en.wikipedia.org/wiki/Luer_taper). When the first and second Luer lock type fluidic connection points are connected to one another, the biosafety valve causes the fluidic inlet to be open and allows the flow in both directions, hence this biosafety valve is a two-way check valve.

Advantageously, the Luer lock type configuration in the first and second fluidic connection points blocks the fastening of the storage and propulsion means and thereby prevents leakages of the fluids contained in said storage and propulsion means.

In a particular embodiment, *the storage and propulsion means for storing and propelling a pre-established amount of second fluid, preferably saline solution for intravenous use, comprising a first fluidic connection point, are a syringe.*

In a particular embodiment, *the connection means of the second end are Luer lock type connection means.* Advantageously, the leakage of fluids when the connection means of the second end are connected with the line of the patient are prevented.

In a particular embodiment, *the main conduit is transparent, flexible, and has no bending memory.*

In a particular embodiment, *the main conduit comprises an antibacterial air intake located between the first end and the drip chamber.* More particularly, this antibacterial air intake comprises a hydrophobic filter which is responsible for the air inlet to be antibacterial, that is, the air intake with the hydrophobic filter is configured for the air entering the device if needed to not allow the passage of any type of bacterium, resulting in an antibacterial filter. The air intake is used in cases where the passage of the first fluid through the main conduit is hindered or even stopped because of a vacuum situation that the device itself may create. For example, sometimes when the first fluid is being administered to the patient, the flow of this first fluid slows down and may even stop. In this situation, the antibacterial air intake is opened for air to enter the main conduit and for the infusion to resume, guaranteeing that the air that enters is free of bacteria.

In a particular embodiment, *the drip chamber comprises a particle filter to establish a barrier to prevent, in the operative mode, the passage of air to the second end of the main conduit.* More particularly, the particle filter is a hydrophilic filter. The arrangement of this particle filter advantageously keeps the system air-free, preventing the first fluid from not moving forward through the main conduit once this first fluid has run out in the storage element.

In a particular embodiment, *the device further comprises a flow regulator interposed in a section of the main conduit preferably between the three-way cock and the second end of the main conduit.* More particularly, the flow regulator is proximal to the three-way cock. This flow regulator is configured to regulate the drip rate of the fluid being administered to the patient. Typically, this type of regulator is modified by watching the drip rate until it reaches the desired value.

In a more particularembodiment, *the flow regulator comprises a "roller clamp" whereby the drip rate of the fluid being administered to the patient is fixed.* More particularly, the "roller clamp" is a wheel. This wheel is displaced vertically over a guide wedging against the tubing in one of the directions. In this way, by rolling it towards the first end of the main conduit, the rate of the fluid increases because the main tubing or conduit is left free of pressure. In contrast, by rolling the wheel towards the second end of the main conduit, the rate of the fluid slows down because the main tubing or conduit is choked by the action of the wheel, even being able to stop the flow altogether.

### DESCRIPTION OF THE DRAWINGS

These and other features and advantages of the invention will be shown more clearly based on the following detailed description of a preferred embodiment, given solely by way of illustrative and non-limiting example, in reference to the attached figures.
Figure 1 shows a schematic view of an infusion device in the operative mode according to an embodiment of the present invention.
Figure 2 shows a schematic view of an infusion device in the operative mode according to another embodiment of the present invention.
Figure 3 shows a schematic view of an infusion device in the operative mode according to another embodiment of the present invention.
Figure 4 shows a schematic view of an infusion device in the operative mode according to another embodiment of the present invention.

### DETAILED DISCLOSURE OF THE INVENTION

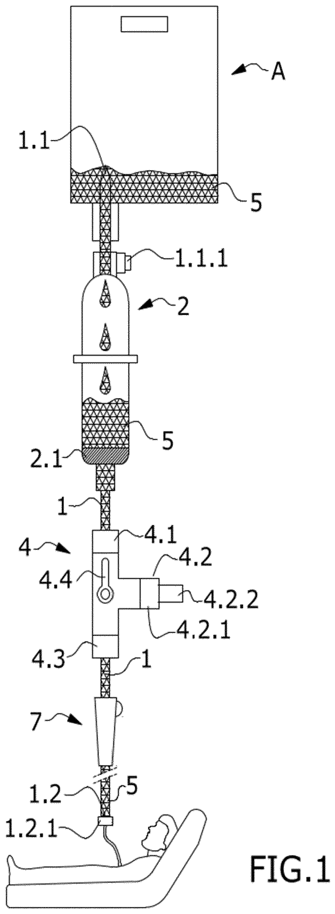

Figures 1 to 4 describe the present gravity infusion device connected to a storage element (A) and to a line connecting with the patient. The storage element (A) initially houses a first fluid (5), or drug, for infusion through the intravenous line of a patient. In particular, the storage element is a bag (A).

The infusion device connects at a first end (1.1) of a main conduit (1) with the bag (A) and at a second end (1.2) of the main conduit (1) with a line. The first end (1.1) is a perforating element of a piercing device type configured to be inserted into the bag (A). The second end (1.2) comprises connection means (1.2.1) which are those that, in the operative mode, connect with the line through which the drug (5) is administered to the patient. In particular, the main conduit (1) is transparent, flexible, and has no bending memory, and the connection means (1.2.1) arranged at the second end (1.2) are Luer lock type connection means.

The infusion device in turn comprises a drip chamber (2) interposed in a section of the main conduit (1) closer to the first end (1.1) of the main conduit (1) than to the second end (1.2). This drip chamber (2) is configured to continuously see the drip rate of the drug (5) that will be administered to the patient.

The drip chamber (2) in turn comprises a particle filter (2.1) which acts like a barrier against the passage of fluid once the first fluid (5) in the bag (A) or storage element has run out to prevent air from passing to the rest of section of the conduit and reaching the second end (1.2). In particular, this particle filter (2.1) is located at the lower base of the drip chamber (2), as can be seen in the schematic figures.

After the drip chamber (2), the infusion device comprises a three-way cock (4) interposed in a section of the main conduit (1) located between the drip chamber (2) and the second end (1.2) of the main conduit (1), the three-way cock (4) mainly being made up of three ports (4.1, 4.2, 4.3). The three-way cock (4) is configured to control the passage of fluid to the second end (1.2) of the main conduit (1), that is, to selectively control the passage of the drug (5) or of a second fluid (6).

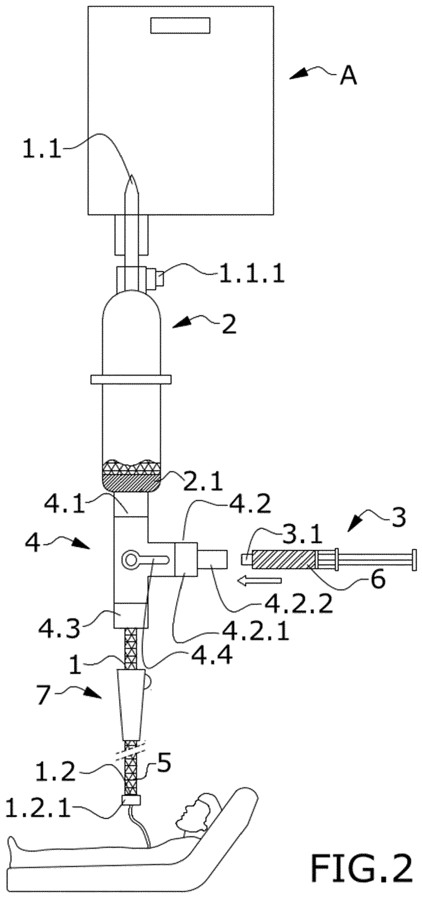

A first port (4.1) of the three-way cock (4) is in fluidic communication with the drip chamber (2), this first port being the inlet port (4.1) of the drug (5). The three-way cock (4) can be connected directly to the drip chamber (2) as shown in Figure 2, where it can be seen how the first inlet port (4.1) is connected to the lower base of the drip chamber (2). In contrast, the three-way cock (4) can be connected indirectly to the drip chamber (2) by means of a section of the main conduit (1) as observed in Figures 1, 3 and 4, such that the first inlet port (4.1) is connected to a section of the main conduit (1), this section of the main conduit (1) being arranged between the drip chamber (2) and the first inlet port (4.1) of the three-way cock (4).

A second inlet port (4.2) of a second fluid (6) or saline solution for intravenous use. The second port (4.2) is configured to be connected with a storage and propulsion means (3) (as shown in Figures 2-4) which house the saline solution (6) therein.

The infusion device further comprises a storage and propulsion means (3) suitable for housing a pre-established amount of the saline solution (6) (as shown in Figures 2 to 4). In this particular example, the storage and propulsion means are a syringe (3) comprising a first fluidic connection point (3.1) configured to connect with a second fluidic connection point (4.2.2) which is in the second port (4.2) of the three-way cock (4).

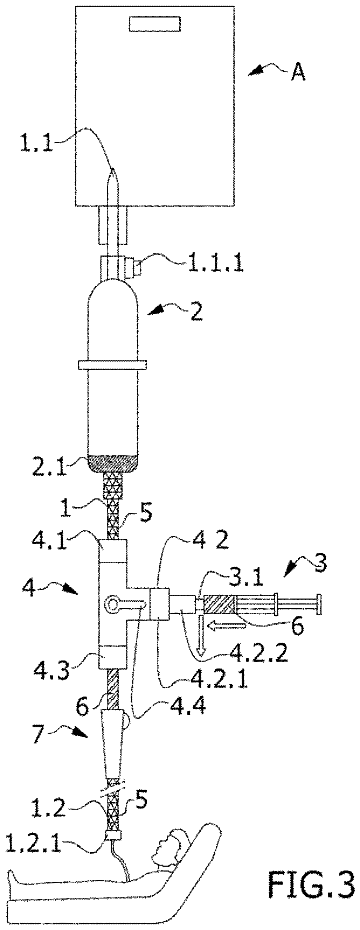

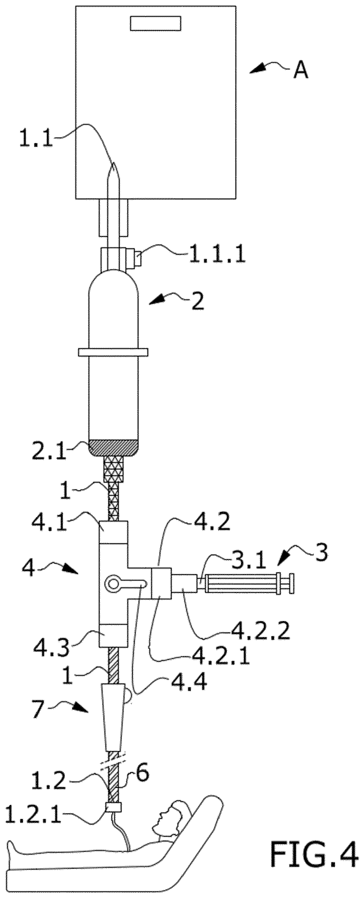

The second inlet port (4.2) in turn comprises a biosafety valve (4.2.1) that controls the entry of the saline solution (6) from the syringe (3) towards the three-way cock (4). The biosafety valve (4.2.1) is open when the first fluidic connection point (3.1) of the syringe (3) is connected with the second connection point (4.2.2) of the second inlet port (4.2) (as shown in Figures 3 and 4), and is closed when there is no connection between the first fluidic connection point (3.1) and second fluidic connection point (4.2.2) (as shown in Figure 2). In particular, the biosafety valve (4.2.1) is arranged between the three-way cock (4) and the second fluidic connection point (4.2.2). According to one example, both fluidic connection points (3.1, 4.2.2) are Luer lock type fluidic connection points.

Lastly, a third port (4.3) in fluidic communication with a section of the main conduit (1) extends from the three-way cock (4) to the second end (1.2). This third port (4.3) is an outlet port both for the drug (5) and for the saline solution (6).

The infusion device in turn comprises an antibacterial air intake (1.1.1) which is located between the first end (1.1) of the main conduit (1) and the drip chamber (2).

The infusion device in turn comprises a flow regulator (7) which is located in a section of the main conduit (1); in this embodiment, it is positioned between the third port (4.3) of the three-way cock (4) and the second end (1.2) of the main conduit. In particular, the flow regulator (7) is arranged proximal to this third port (4.3) of the three-way cock (4). This flow regulator (7) establishes the drip rate of the drug (5) through the main conduit (1) to its second end (1.2).

The three-way cock (4) has two operative positions, a first and a second. The first operative position is shown in Figures 1 and 2, where the first port (4.1) and the third port (4.3) are in fluidic communication. That is, the three-way cock (4) allows the passage of the drug (5) coming from the drip chamber (2) to the second end (1.2) of the main conduit (1) to be administered to the patient. While the three-way cock (4) is in its first operative position, the drug (5) is administered to the patient by means of gravity infusion. In this first operative position, the second port (4.2) is closed, thereby preventing the entry of fluids through this second port (4.2).

On the other hand, the second operative position is shown in Figures 3 and 4, where the second port (4.2) and the third port (4.3) are in fluidic communication. In this case, the three-way cock (4) allows the passage of the saline solution (6) coming from the syringe (3) to the second end (1.2) in order to thus displace the trace of drug (5) that is left housed in the section of the main conduit (1) existing between the third port (4.3) and the second end (1.2). In this second operative position, the first port (4.1) is closed, which forces the saline solution (6) to flow to the second end (1.2) of the main conduit (1), thereby displacing the trace of drug (5). Discarding the drug (5) is thereby prevented, and the largest possible amount of the dose of the drug (5) that had previously been prescribed being administered to the patient is furthermore ensured. In other words, traces of drug (5) are prevented from being left in the infusion device or the amount of such traces are the lowest possible.

The three-way cock (4) includes a tab (4.4) which is arranged in a direction thereby specifically indicating which of the two inlet ports (4.1, 4.2) is open and which one is not. For example, in Figure 1, the tab is arranged towards the first port (4.1), which means that it is open and the passage of the drug (5) to the second end (1.2) is allowed while the second inlet port (4.2) is closed. On the other hand, in Figures 2, 3 and 4 the tab is arranged towards the second inlet port (4.2), indicating that it is open, which allows the passage of the saline solution (6) to the second end (1.2) while the first port (4.1) is closed. That is, in these examples the tab (4.4) shows the origin of the flow going through the three-way cock (4).

In the operative mode, Figure 1 shows the gravity infusion of the drug (5) into the patient since this drug (5) flows along the infusion device to the second end (1.2) of the main conduit (1).

Figure 2 shows how the bag (A) has been emptied of drug (5) and how traces of this same drug are housed in the drip chamber (2), pending infusion, as well as traces from the drip chamber (2) to the second end (1.2) of the main conduit (1). To prevent there being traces of drug (5) between the drip chamber (2) and the remaining section of the main conduit (1) up to its second end (1.2) once the infusion has ended, or at least the amount of these traces is minimized, this embodiment of the invention proposes the administration of the saline solution (6) through the second inlet port (4.2) of the three-way cock as explained above. In this Figure 2, it can particularly be seen how the syringe (3) approaches the second inlet port (4.2) of the three-way cock (4) to deliver the saline solution (6) to the device so that no traces of drug (5) are left in the main tubing or conduit (1) between the drip chamber (2) and the second end (1.2) of the main conduit (1). The horizontal arrow observed in Figure 2 shows the direction in which the syringe (3) will provide the saline solution (6) through the second port (4.2).

Figure 3 shows how the saline solution (6) is administered to the three-way cock (4) by means of the syringe (3) and it can be observed how this saline solution (6) flows through the three-way cock (4) and also through the final section of the main conduit (1), thus displacing the traces of drug (5) to the second end (1.2) of the main conduit (1). The horizontal and vertical arrows observed in Figure 3 show the direction of fluid to be followed by the saline solution (6).

In Figure 4, it can be seen how in the final section of the main conduit (1), there is no longer any traces of drug (5) as a result of the administration of the saline solution (6) which has reached the second end (1.2) of the main conduit (1). In this figure, it can also be seen how the syringe (3) is empty since it has provided the total pre-established amount of saline solution (6) housed therein, and now this pre-established amount of saline solution (6) is arranged in the three-way cock (4) and the final section of the main conduit (1) up to the second end (1.2).

In an example which is not shown in the figures, the three-way cock (4) comprises three wings which togethergive rise to a T-shaped configuration. In this case, the wings having an orientation coinciding with the orientation of a port (4.1, 4.2, 4.3) of the three-way cock indicates the opening of said port. To change the position of the three-way cock (4), this T-shaped configuration is rotated to thus regulate the passage of fluid of interest at all times.

## Claims

1. A gravity infusion device suitable for the intravenous infusion of a first fluid (5), wherein the device comprises:
- a main conduit (1), with a first end (1.1) intended for being fed by a storage element (A) for storing the first fluid (5) to be infused and a second end (1.2) with connection means (1.2.1) to be connected to a line for delivering, in the operative mode, the first fluid (5) to a patient;
- a drip chamber (2) interposed in a section of the main conduit (1) proximal to the first end (1.1);
- storage and propulsion means (3) for storing and propelling a pre-established amount of a second fluid (6), preferably saline solution for intravenous use, comprising a first fluidic connection point (3.1);
- a three-way cock (4) interposed in a section of the main conduit (1) between the drip chamber (2) and the second end (1.2), wherein the three-way cock (4) comprises:
∘ a first inlet port (4.1) fluidically connected with the drip chamber (2);
∘ a second inlet port (4.2) further comprising
▪ a second fluidic connection point (4.2.2); and
▪ a biosafety valve (4.2.1), that is, the valve is open if the second port (4.2) is fluidically connected with the first fluidic connection point (3.1) and closed if it is not connected, the biosafety valve (4.2.1) being placed before the second fluidic connection point (4.2.2) and configured to allow the entry of the second fluid (6) from the storage and propulsion means (3) to the three-way cock (4);
∘ a third outlet port (4.3) fluidically connected with a section of the main conduit (1) extending up to the second end (1.2);
wherein
a) the three-way cock (4) comprises two operative positions:
a first operative position in which the first port (4.1) and the third port (4.3) are fluidically communicated while the second port (4.2) is fluidically closed, and
a second operative position in which the first port (4.1) is fluidically closed, and
the second port (4.2) and the third port (4.3) are fluidically communicated;
b) the first fluidic connection point (3.1) is configured to be coupled to the second fluidic connection point (4.2.2) such that, in the operative mode, the propulsion of the second fluid (6) from the storage and propulsion means (3) displaces at least part of the first fluid (5) stored in the section of the main conduit (1) arranged between the three-way cock (4) and the second end (1.2).

2. The device according to claim 1, wherein the first end (1.1) is a perforating element configured to be inserted into the storage element (A) which is preferably a bottle or bag of the first fluid (5) intended for being infused in a patient.

3. The device according to claim 1 or 2, wherein the three-way cock (4) comprises three wings providing with one another a T shape, and wherein the wings having an orientation coinciding with the orientation of a connection port (4.1, 4.2, 4.3) indicate the opening of said port.

4. The device according to any of the preceding claims, wherein the first fluidic connection point (3.1) and second fluidic connection point (4.2.2) are "Luer lock" type fluidic connection points.

5. The device according to any of the preceding claims, wherein the storage and propulsion means (3) for storing and propelling a pre-established amount of second fluid (6), preferably saline solution for intravenous use, comprising a first fluidic connection point (3.1), are a syringe.

6. The device according to any of the preceding claims, wherein the first fluid (5) is preferably a drug.

7. The device according to any of the preceding claims, wherein the connection means (1.2.1) of the second end (1.2) are "Luer lock" type connection means.

8. The device according to any of the claims, wherein the main conduit (1) is transparent, flexible, and has no bending memory.

9. The device according to any of the preceding claims, wherein the main conduit (1) comprises an antibacterial air intake (1.1.1) located between the first end (1.1) and the drip chamber (2).

10. The device according to any of the preceding claims, wherein the drip chamber (2) comprises a particle filter (2.1) to establish a barrier to prevent, in the operative mode, the passage of air to the second end (1.2) of the main conduit (1).

11. The device according to any of the preceding claims, wherein the device further comprises a flow regulator (7) interposed in a section of the main conduit (1) preferably between the three-way cock (4) and the second end (1.2) of the main conduit.
